# EUROPEAN PATENT APPLICATION

(11) **EP 2 165 668 A2**
(43) Date of publication of application: **24.03.2010**
(21) Application number: 09170951.9
(22) Date of filing: 22.09.2009
(51) Int. Cl.: A61B 17/88, A61F 2/46

(54) **Surgical insertion tool and guide system**

(30) Priority: 22.09.2008 US 234857
(71) Applicant: RSB Spine, LLC, Cleveland, OH 44114 (US)
(72) Inventor: Whiteaker, Mark T., Rocky River, OH 44116 (US); Bray, Robert S., Studio City, CA 91604-4170 (US)
(74) Representative: BOVARD AG

(57) **Abstract**

An insertion tool (10) has a curved fastener guide (14a;14b) for allowing a user to safely insert bone fasteners (42) and associated tools at the surgical site for securing an implant device (40) to a bone body. The insertion tool has an elongate cannula (12) and attachment shaft (18) coupled to the distal end of the fastener guide. The attachment shaft can be docked to an implant device and the insertion tool can be used to locate the implant device at the surgical site. The fastener guide can be aligned with fastener holes in the implant device for accurate placement of the bone fasteners.

## Description

### FIELD OF THE INVENTION

The present invention relates generally to insertion tools for assisting in surgery, and more particularly to an insertion tool and guide system for introducing bone fasteners and implant devices to a surgical site.

### BACKGROUND OF THE INVENTION

In the art of spinal surgery, an increasing number of fixation implant devices are being developed. Fixation implant devices often utilize bone fasteners (e.g., screws) to fix the implant device to the spine and retain the device. The bone screws can be inserted into spinal vertebra at varying angles of entry to allow for settling and movement of the vertebra. For example, the bone screws can be angled from the anterior surface of the implant device into the vertebral endplates above and below the device.

Depending on the approach, insertion of bone screws can present difficult angles for the surgeon unless a large incision is made to accommodate for angling long fastener tools. A small incision is preferable; however the risk of damaging vital organs is increased if multiple fastener tools are used to angle bone screws into bone bodies. In spinal surgery, the spine can be accessed either anteriorly or posteriorly. In an anterior approach, the surgeon must manoeuvre around vital neural, vascular and visceral structures to implant a fixation device. Insertion of bone screws past such structures increases the duration and degree of difficulty of the surgery.

### SUMMARY OF THE INVENTION

An insertion tool includes an elongate cannula having a proximal end and a distal end, and a fastener guide having a proximal end and a distal end. The distal end of the fastener guide can be coupled to the distal end of the elongate cannula. A portion of the fastener guide can be curved, and the fastener guide can have an opening at its proximal end for receiving a bone fastener.

According to different embodiments, the bone fastener can be attachable to a driver device. The elongate cannula can have a passage there through for receiving an attachment shaft. The attachment shaft can have a proximal end and a distal end, wherein this attachment shaft has attachment means secured to the distal end of the attachment shaft. The attachment means can be suitable for attaching an implant device to the distal end of the attachment shaft. The attachment shaft can extend through the elongate cannula. The attachment shaft can also be rotatable within the elongate cannula. The insertion tool can further include a connector for coupling the distal end of the fastener guide to the distal end of the elongate cannula. The distal end of the fastener guide can also be coupled at an angle relative to the elongate cannula. This angle can be in the range of 15 to 60 degrees. The proximal end of the fastener guide can be substantially parallel with the proximal end of the elongate cannula. The connector can include a through hole for receiving an attachment shaft. The attachment shaft can also extend through the elongate cannula and the through hole in the connector. The insertion tool can further include an attachment means secured to one end of the attachment shaft. Finally, a portion of the attachment means can also extend outward beyond the through hole in the connector.

An insertion tool for implanting an implant device has at least one fastener hole, the insertion tool including a curved fastener guide having a proximal end and a distal end, the distal end of the fastener guide being aligned with the fastener hole of the implant device. The insertion tool further includes an elongate cannula and an attachment shaft. The elongate cannula has a proximal end and a distal end, wherein the elongate cannula further has a passage there through for receiving the attachment shaft. The attachment shaft extends through the elongate cannula and is docked to the implant device to be implanted.

According to different embodiments, the curved fastener guide can have an opening at the proximal end for receiving a bone fastener. The distal end of the elongate cannula and the distal end of the curved fastener guide can be coupled together. The distal end of the curved fastener guide can be coupled at an angle relative to the distal end of the elongate cannula, which can be in the range of 15 to 60 degrees. The elongate cannula can be straight.

An insertion tool includes an attachment shaft to be held by a user, wherein the attachment shaft has a proximal end and a distal end, where the attachment shaft includes a handle at the proximal end and an attachment means adapted to mate with an implant device at the distal end. The insertion tool further includes a fastener guide having a proximal end and a distal end, wherein the fastener guide is curved such that the distal end of the fastener guide is angled with respect to the proximal end of the fastener guide.

According to different embodiments, the fastener guide can have a passage there through for receiving a fastener tool. The fastener guide can be a hollow tube having a curved portion. The fastener guide can also be rigid. The attachment shaft can be straight and rigid. The fastener guide can also have a length and the attachment shaft can have a length, wherein the length of the fastener guide can be at least 50% of the length of the attachment shaft. The length of the fastener guide can also be at least 75% of the length of the attachment shaft. The fastener guide can be coupled to the attachment shaft. The proximal end of the fastener guide can also be substantially parallel with the proximal end of the attachment guide. The distal end of the fastener guide can be coupled at an angle relative to the attachment shaft. The proximal end of the attachment shaft and the proximal end of the fastener guide can also be dimensioned and configured to be outside of a patient's body during an anterior approach surgical procedure on the spine.

The following description and the annexed drawings set forth details of certain illustrative aspects of the invention. These aspects are indicative, however, of but a few of the various ways in which the principles of the invention may be employed and the present invention is intended to include all such aspects and their equivalents. Other objects, advantages and novel features of the invention will become apparent from the following detailed description of the example embodiments when considered in conjunction with the drawings.

### BRIEF DESCRIPTION OF THE DRAWINGS

The foregoing and other features and advantages of the present invention will become apparent to those skilled in the art to which the present invention relates upon reading the following description with reference to the accompanying drawings.
Figure 1 is a perspective view of an insertion tool according to one example embodiment of the present invention.
Figure 2 is a side view of the insertion tool according to the example embodiment shown in Figure 1.
Figure 3 is a reverse angle cross-section view of the insertion tool as shown in Figure 2.
Figure 4 is a front view of the insertion tool according to the example embodiment shown in Figure 1.
Figure 5 is a rear view of the insertion tool according to the example embodiment shown in Figure 1.
Figure 6 is a bottom view of the insertion tool according to the example embodiment shown in Figure 1.
Figure 7 is a top view of the insertion tool according to the example embodiment shown in Figure 1.
Figure 8 is a side perspective view of the example insertion tool attached to an implant device according to one aspect of the present invention.
Figure 9 is an enlarged view of a portion of the example insertion tool attached to the implant device of Figure 8.
Figure 10 is a perspective view of a portion of the example insertion tool detached from an implant device positioned between two model vertebrae according to one aspect of the present invention.

### DESCRIPTION OF EXAMPLE EMBODIMENTS

The present invention will now be described with reference to the drawings, wherein like reference numerals are used to refer to similar elements throughout. It is also to be appreciated that the various drawings are not necessarily drawn to scale from one figure to another nor inside a given figure, and in particular that the size of the components are arbitrarily drawn for facilitating the understanding of the drawings. In the following description, for purposes of explanation, numerous specific details are set forth in order to provide a thorough understanding of the present invention. For example, in the description that follows, when a range, such as 15 to 60 (or 15-60), is given, this means preferably at least 15 and, separately and independently, preferably not more than 60. It may be evident, however, that the present invention can be practiced without these specific details. Additionally, other embodiments of the invention are possible and the invention is capable of being practiced and carried out in ways other than as described. The terminology and phraseology used in describing the invention is employed for the purpose of promoting an understanding of the invention and should not be taken as limiting.

Various aspects of the present invention are described herein and configured to assist a user during the delivery of an implant device, such as a fixation device or spinal implant to be located between two vertebrae. In one embodiment, an insertion tool including a curved fastener guide and an elongate cannula having an attachment shaft extending there through can be employed to insert an implant device at a desired location and insert bone fasteners into a bone body to secure the implant device in place. The curved fastener guide can be aligned with fastener holes in the implant device to allow for accurate placement of bone fasteners at various angles.

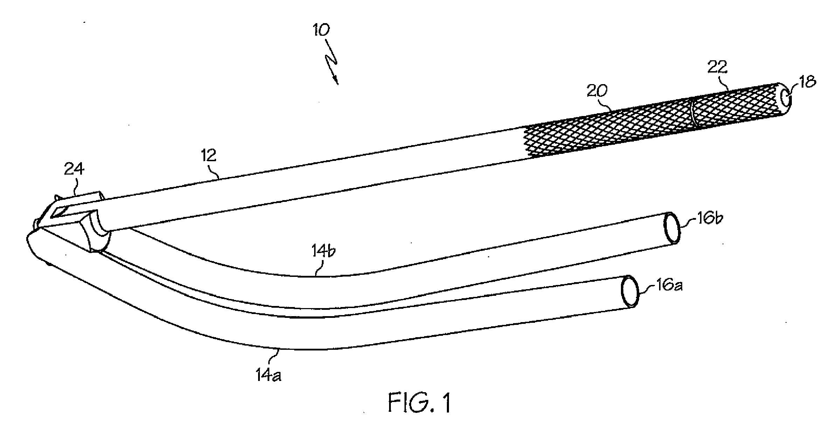

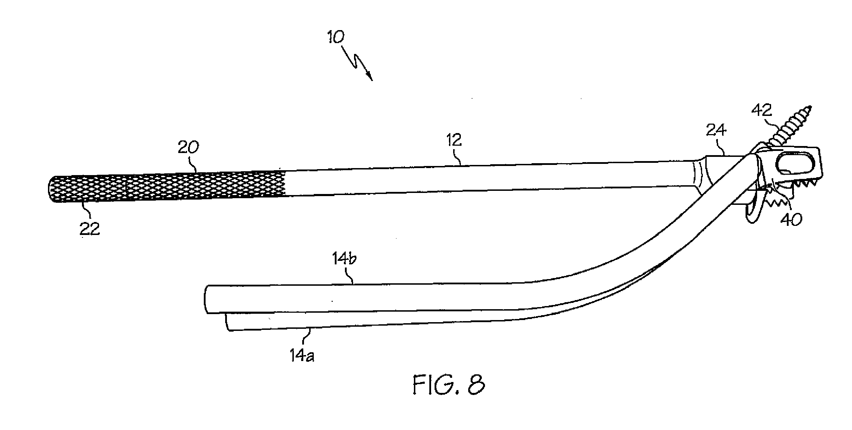

Turning to the Figures, Fig. 1 shows an example insertion tool 10 that is adapted for use with implant devices, such as the implant device 40 as shown in Figs. 8 through 10. While the insertion tool 10 is described and shown herein in conjunction with implant devices 40 capable of being fixed to a bone body at the surgical site, it is contemplated that the insertion tool 10 may be used in conjunction with other types of implant devices that can be used on any part of the body. As shown, the insertion tool 10 generally includes an elongate cannula 12, at least one fastener guide 14a, 14b, an attachment shaft 18 and a connector 24. In general, a user operates the insertion tool 10 by grabbing the attachment shaft 18, at the proximal end, and aligning the opposing end up with an implant device having one or more fastener holes for receiving a bone fastener. The implant device can be attached to the distal end of the shaft 18 such that the fastener guides 14a, 14b of the insertion tool 10 are aligned with the fastener holes in the implant device. In this position, the openings at the distal end of the fastener guides 14a, 14b are in register with the fastener holes in the implant device. The user then inserts the implant device and corresponding end of the insertion tool 10 attached to the device into an incision in a patient's skin. The user aligns the implant device at the targeted bone site and sets the device into the desired position and the fastener bore holes are arranged to face, optionally at an angle, the bone body or bodies that the implant device may be fixed to. The user can then use a fastener tool, such as a drill and drill bit or centre punch on a flexible shaft, to create fastener insertion holes in the bone body by inserting a drill through the curved fastener guides that are aligned with the fastener holes in the implant device. As used herein, the fastener tools generally can be flexible or have a flexible shaft or potion that allows for bending through the fastener guides 14a, 14b. Optionally, the fastener tools can be rigid or have a rigid shaft. In this case, the fastener tool can be adapted for formed in a shape that corresponds to the curved fastener guides 14a, 14b. Once the desired holes are drilled into the bone body, and optionally tapped, the drill can be retracted from the fastener guide and a driver device having a bone fastener at one end can be inserted into the fastener guide and moved there through to the bone body. The driver is used to screw or insert the bone fastener through the fastener hole in the implant device and into the desired bone body having the drilled hole. The implant device is fixed or screwed into place on one or more bone bodies, and the implant device can be detached from the attachment shaft and the insertion tool 10 can be removed from the patient's body through the incision.

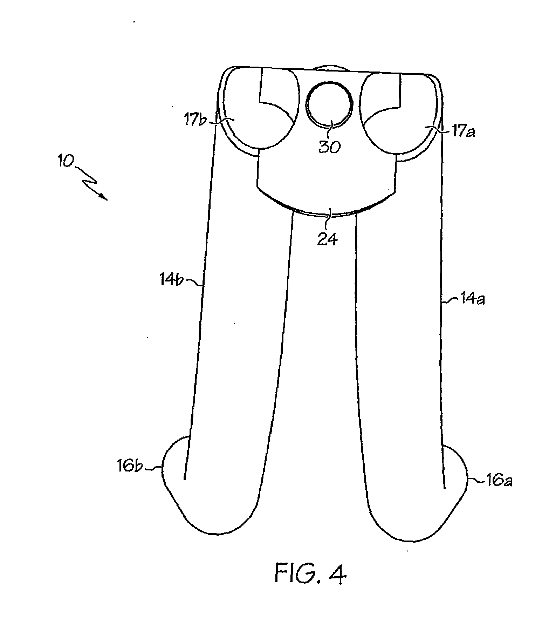

In an alternative use for inserting the bone fasteners, a user can insert a bone fastener into the distal end of the fastener guides 14a, 14b prior to inserting the insertion tool 10 having an implant device secured thereto into a patient's body. To minimize risk of patient injury, the bone fasteners can be positioned in the distal end of the fastener guides 14a, 14b such that the bone fastener, or a portion thereof, does not protrude or extend outward from the distal openings 17a, 17b (as shown in Fig. 4). To engage the bone fasteners, a fastener tool, such as a driver, can be inserted into the proximal opening 16a, 16b of the fastener guides 14a, 14b and moved along the curved length of the fastener guides until the fastener tool comes into contact with the bone fastener. The bone fastener, via the fastener tool, can then be inserted into the bone body to fix the implant device. Inserting the bone fastener in the distal end of the fastener guides 14a, 14b can minimize the required inner diameter of the fastener guides 14a, 14b that would otherwise be sized to accommodate the dimensions of the bone fastener and allow the bone fastener to move through the curved portion of the fastener guides 14a, 14b.

As used herein, the phrase "bone body" 50 is intended to include an individual bone as well as fragments or portions of a bone. For example, the bone body 50 can be a vertebra of the spine. Also as used herein, a bone fastener 42 can include a bone screw or securing pin, a plurality of which can be used for securing or fixing an implant device 40 to adjacent bone bodies 50. The bone fasteners 42 can be configured to pass through the fastener holes in an implant device and can have pointed ends which include a cutting flute on the tip. The cutting flute at the tip of the bone fastener 42 allows the fastener to be self-drilling or self-tapping. Thus, the use of a bone fastener 42 having a self-drilling or self-tapping tip makes the use of a drill or centre punch optional. The bone fastener 42 can be made of any suitable material, such as stainless steel, titanium or a titanium alloy, a radiolucent material, a radiopaque material, or combinations thereof.

As shown in Fig. 1, the two curved fastener guides 14a, 14b are coupled to the elongate cannula 12 and attachment shaft 18 with a connector 24. The fastener guides 14a, 14b provide a protective shield in which fastener-type tools can extend or slide along the length of the guides for desirably penetrating a bone body through an implant device. The fastener guides 14a, 14b each have a proximal end and a distal end, wherein the distal end of each guide 14a, 14b is coupled to a connector 24. Fastener guides 14a, 14b have a length that travels from the connector 24 and curves to extend outward in a direction that is substantially parallel to the attachment shaft 18 and elongate cannula 12 of the insertion tool 10. Fastener guides 14a, 14b have openings 16a, 16b at each respective proximal end and openings (not shown in Fig. 1) at each respective distal end. The openings at each end of the guides provide access to the passage extending through the centre of the guides. As shown in the presented example, the proximal end openings 16a, 16b have a circular shape, although other suitable shapes may be used. The fastener guides 14a, 14b can be hollow tubes having a curved portion such that the guides 14a, 14b define circular passages for receiving a bone fastener, of fastener tool. Although the passage extending through the guides is generally circular in cross section, any cross section shape can be used to receive a drill or fastener driver that may be employed during a surgical procedure. The fastener guides 14a, 14b may have an inside diameter suitable for receiving a bone fastener and various fastener tools, such as those used for fixation and/or bone plate alignment procedures. For example, the fastener guides 14a, 14b can have an inner diameter from about 3 mm to 9 mm, or about 7 mm. The fastener guides 14a, 14b can be made from any suitable material, such as metal, stainless steel, titanium, alloys such as titanium alloy, and the like. As discussed herein, the components of the insertion tool 10 can be sterilized, passivated and electropolished as known in the art. The surfaces of the components, other than the gripping textures described below, are burr-free to allow for a smooth surface that moves easily against a patient's skin and internal tissue.

As can be seen in Fig. 1, the fastener guides 14a, 14b have a length that can be measured from the proximal end to the distal end of the guides. Similarly, the elongate cannula 12 and attachment shaft 18 have lengths that can be measured from their respective proximal and distal ends. The fastener guides 14a, 14b can have lengths that are in the range of 50% to 100% of the length of the attachment shaft 18 and/or the length of the elongate cannula 12. In one embodiment, the guides 14a, 14b can have a length that is at least 50% or at least 75% of the length of the attachment shaft 18. The fastener guides 14a, 14b can be 75 mm to 250 mm, or about 180 mm. As shown in Figs. 1 through 3, the curved fastener guides 14a, 14b can have a substantially straight portion extending from one end of the curved portion to the proximal end of the fastener guides 14a, 14b. The substantially straight portion from the curved portion to the proximal ends of the fastener guides 14a, 14b can vary in length, and for example, the substantially straight portion can be 1% to 99% of the overall length of the fastener guides 14a, 14b.

The length of the fastener guides, 14a, 14b as compared to the elongate cannula 12 or attachment shaft 18, beneficially allows a user or surgeon to locate the distal end of the insertion tool 10 having an implant device secured thereto at a surgical site without harming or damaging tissue and organs between the site and the patient's skin. For example, in an anterior approach to the spine, internal abdominal organs and other soft tissue may be between the skin incision and spine, and therefore sharp instruments, such as fastener tools and bone fasteners would need to travel through the patient's body to reach the desired spinal vertebra at the surgical site. The fastener guides 14a, 14b are of sufficient length to ensure that the proximal ends of the guides 14a, 14b, along with the proximal end of the attachment shaft 18, extend outside the patient's skin during surgical procedures. Thus fastener tools can then be easily inserted into the guides 14a, 14b at openings in the proximal end without contacting the patient's skin and internal organs or soft tissue. Furthermore, the length of the guides, being greater than the distance between the surgical site and the patient's skin surface, at the incision reduces bone debris that may be deposited in the body cavity during a securing procedure for a fixation device or other implant device. This allows for a clean and safe surgical procedure with less risk of complications, such as tissue damage or infection. To accommodate the use of various fastener tools, the proximal ends of the fastener guides 14a, 14b can be spaced apart from each other and/or the elongate cannula 12 and attachment shaft 18 or handle 22. Adequate spacing between the portions of the fastener guides 14a, 14b, elongate cannula 12 and attachment shaft 18 extending outside of a patient's body during a surgical procedure accommodates hand movements of the user, rotation of the fastener tools and the hand engagement with the various size and dimension of handles of the fastener tools.

As further seen in Fig. 1, the fastener guides 14a, 14b can have a curved portion between the proximal end and distal end such that a centre portion of the guides 14a, 14b located between the ends can be curved. The curved potion of the guides allows the proximal end and the distal end of each guide 14a, 14b to be angled relative to one another. The curved portion allows the end of the guides 14a, 14b be angled to the desired bone fastener insertion angle of an implant device. For example, an implant device, as discussed above, can have one or more fastener holes for receiving a bone fastener used to fix or secure the implant device to a bone body. A bone fastener, such as a bone screw, can be angled through such a fastener hole, or in the alternative, the fastener hole in the implant device can be positioned at angle in the implant device, such as an angled fastener hole. In either case, it may be necessary to insert the bone fastener into a bone body at angle. The curved portion of the guides 14a, 14b allows the angled (with respect to the proximal end) distal end of the guides to properly align with angled fastener holes such that a bone fastener can be accurately inserted into a bone body by sliding the bone fastener and/or fastener tool through the guides 14a, 14b. The angled distal end of the of guides 14a, 14b can optionally be mated to flat, non-angled fastener holes so a respective bone fastener can be inserted into a bone body at any angle at which the distal end of the guide is mated.

The curvature of the fastener guides 14a, 14b allows a user to insert fasteners into bone bodies while utilizing a minimal-sized incision in the patient's skin. In other words, if the fastener guides were long straight tubes, and the user wanted to fasten bone screws into a bone body at a 45-degree angle, the guides would extend outward at a 45-degree angle with respect to the attachment shaft 18, and create significant distance between the proximal ends of the shaft 18 and straight guides. Thus, such large straight guides would require a large incision to accommodate the distance between the proximal ends of the guides and shaft. The curved fastener guides 14a, 14b, which can angle back to be substantially parallel with the attachment shaft 18 and/or elongate cannula 12, minimize the distance at the respective proximal ends and reduce the size of incision needed in the patient's skin. In cases where the distance from the incision to the surgical site is significant, such as with an anterior approach in a lumbar spine surgery or that encountered with larger patients, the curved fastener guides 14a, 14b are ideally suited since the size and dimensions of the incision can be minimized while allowing bone fasteners to be located at many desirable angles. Furthermore, in certain surgical procedures, such spinal surgery accessing the lower cervical spine, a long, angled straight fastener guide can impinge on a patient's body and rib cage. The curved fastener guides 14a, 14b allow a user to avoid the rib cage or push against it when accessing a lower cervical surgical site.

The fastener guides 14a, 14b are positioned at an angle relative to the attachment shaft 18 by coupling the guides to the elongate cannula 12 and or attachment shaft 18. The guides are coupled to the elongate cannula 12 and shaft 18 by a connector 24, such as metal block tooled to have recessed cavities corresponding to the shape and dimensions of the guides and cannula. The connector 24 can be made from any suitable material, such as metal, stainless steel, titanium, alloys such as titanium alloy, and the like. The fastener guides 14a, 14b, and elongate cannula 12 can be permanently attached to the connector 24 by conventional means, such as by welding or using an adhesive. The connector 24, and the components attached thereto, such as fastener guides 14a, 14b and the elongate cannula 12, can thus be considered to form the insertion tool 10 as an integral tool.

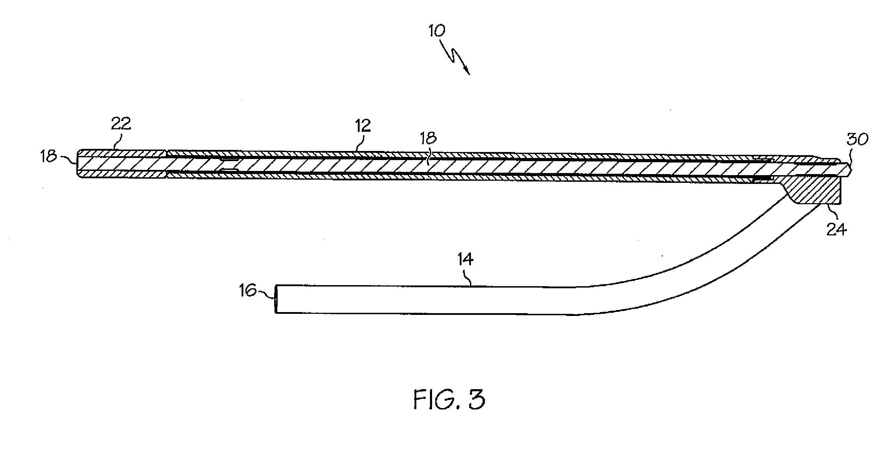

As shown in Fig. 1, and notably in Figs. 3 and 4, the connector 24 is tooled with two partial circular cavities for receiving the distal ends of the guides 14a, 14b. The partial circular cavities are angled to facilitate the angled-mount of the guides such that the guides can align with fastener holes in an implant device. A portion of the fastener guides 14a, 14b extend out of the partial circular cavities. The connector 24 can have a through hole for receiving an end of the elongate cannula 12 and the attachment shaft 18. The through hole in the connector 24 can extend entirely through the connector and can have any suitable shape, such as a tube with a circular cross-section. The elongate cannula 12 can be fitted into a portion of the connector through hole and the distal end of the attachment shaft 18 can extend through the remaining portion of the connector through hole such that a portion of the distal end of the shaft 18 protrudes out of one side of the connector 24. The connector through hole can be sized to have an inner diameter larger than the portion of the attachment shaft 18 passing there through to allow rotation and slidability of the shaft 18 within the through hole. The portion of the connector through hole engaging the elongate cannula 12 can be sized to provide a tight seal between the distal end of the cannula 12 and the connector 24. The distal end of the cannula 12 can be permanently attached to the through hold of the connector 24 with an adhesive or by welding.

Turning to the other components of the insertion tool 10, the elongate cannula 12 can be straight and hollow, and suitably designed to receive an attachment shaft 18 there through. The elongate cannula 12 can be made from any suitable material, such as metal, stainless steel, titanium, alloys such as titanium alloy, and the like. As noted above, the distal end of the elongate cannula 12 can be attached to the connector. The proximal end and the distal end of the cannula 12 are open and allow the attachment shaft 18 to extend there through. The inner diameter of the cannula 12 can be sized to accommodate the attachment shaft 18 and allow shaft to slide and/or rotate within the cannula 12. The inner diameter of the cannula 12 can be 3 mm to 9 mm, or about 4 or 5 mm. The proximal end portion of the cannula 12 can have knurling 20, for example in the form of cross-hatching, ridges or teeth to enhance gripping of the cannula 12 by the hand of a user. In similar fashion, the proximal end portion of the attachment shaft 18 and/or handle can have knurling and the like for enhancing grip. In the case a handle 22 is used, the handle 22 can have various sizes and configurations for accommodating different hand sizes and allowing a user to grip and manipulate the attachment shaft 18 to accurately position an implant device at the surgical site. For example, the handle 22 can be configured as a pistol-type grip (not shown) or other features used to engage or disengage locking mechanisms on the distal end.

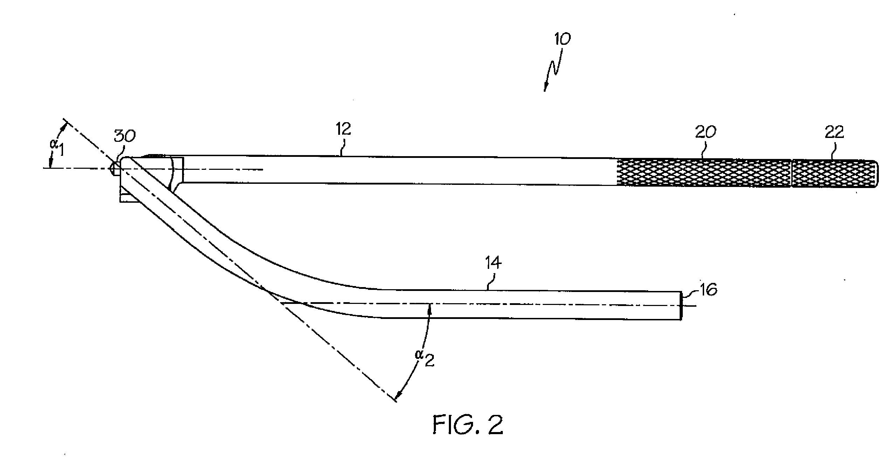

The attachment shaft 18 has a proximal end and a distal end. The attachment shaft 18 can be straight and rigid and be made of any suitable material, such as metal, plastic, stainless steel, titanium, alloys such as titanium alloy, and the like. The proximal end of the attachment shaft 18 can include a handle 22. The handle portion 22 of the shaft 18 can have a larger diameter than the remaining portion of the shaft 18. The larger diameter of the handle portion 22 acts as a stop plate when the shaft 18 is inserted through the elongate cannula 12. Depending of the length of the handle portion 22, the length of the shaft 18 extending through the cannula 12 and/or connector 24 can be selectively controlled such that only the desired amount of the distal end of the shaft 18 extends out of the cannula 12 and/or connector 24. The distal end of the shaft 18 can have an attachment means 30 (not shown) secured thereto. The attachment means 30 is discussed in detail below with regard to Figs. 2 and 4.

In use, the attachment shaft 18 can be inserted into the centre opening of the elongate cannula 12. The elongate cannula 12 can be permanently attached to the connector 24 such that the centre passage of the cannula 12 is aligned and in register with the connector through hole. The shaft 18 extends completely through the cannula 12 and connector through hole in the connector 24. The distal end of the attachment shaft 18 protrudes outward from the connector through hole. An implant device can be attached to the distal end of the shaft extending outward from the connector 24. The portion of the attachment shaft 18 extending out of the connector through hole can have an attachment means 30 secured thereto for mating to or docking an implant device.

As shown in Fig. 2, the distal end of the attachment shaft 18 can have an attachment means 30. The attachment means 30 can include a separate piece secured to the distal end of the shaft 18, such as collared sleeve having a threaded portion. The attachment means 30 can also include molding for forming the distal end of the shaft 18 into a desired shape for mating to an attachment area or cavity of an implant device such that the device can be docked to the shaft 18. For example, the distal end of the attachment shaft 18 can be formed or forged with a smaller diameter than the remaining portion of the shaft 18. The end of the attachment means 30 can be rounded or formed into a point for mating with an implant device. Threads can be formed on a portion of the attachment means in the case the implant device can be docked to the attachment shaft 18 by means of a threaded fitting. In most cases, the attachment means 30 forms the male portion of a female-to-male connection between an implant device and an attachment shaft 18. It is also contemplated that a releasable adhesive can be used to dock an implant device to the distal end of the attachment shaft 18 or attachment means 30.

Figs. 2 through 4 show the attachment means 30 extending outward from a flat face surface of connector 24. The flat face surface of the connector 24 fits with the openings 17a, 17b in the distal ends of the guides 14a, 14b to form a uniform and integral flat face for securing an implant device. The guide openings 17a, 17b form a portion of the flat face of the connector 24 such that the distal end of the insertion tool 10 is flat except for the portion of the attachment shaft 18 and/or attachment means 30 extending outward from the connector 24. The guide openings 17a, 17b attached to the connector are angled relative to the position of the elongate cannula 12 and attachment shaft 18, which are shown in the horizontal position, which can be the 0-degree reference for angle α1 described below. As shown in Fig. 2, the angle formed between the distal end of the guides 14a, 14b and the elongate cannula 12 and/or attachment shaft 18 is represented by the symbol α1. The distal end of the fastener guides 14a, 14b can form various angles, α1, with respect to the cannula 12 and shaft 18. For example, angle α1 can be in the range of 15 to 60 degrees, or about 40 degrees. The angle α1 formed allows a user to insert a bone fastener into a bone body, and optionally through an implant or fixation device, at a desired approach angle. Angle α1 can correspond to the angle of a fastener hole in an implant device, or can allow a user to position a bone fastener at a desired angle through a non-angled fastener hole. In use, angle α1 is predetermined and based on the desired angle of implantation of the bone fastener used to secure an implant device to a bone body.

Fig. 2 also shows another angle, α2, which is formed between the proximal end and the distal end of a fastener guide 14. As shown, the proximal portion of the fastener guide 14 is substantially parallel to the elongate cannula 12 and attachment shaft 18. Moving along the length of the fastener guide 14, from the proximal end to the distal end, guide 14 curves upward towards the elongate cannula 12 and connector 24. The curved portion of the fastener guide 14 angles the distal end of the guide 14 at the desired angle, α1, with respect to the cannula 12 and/or attachment shaft 18. The curved portion of the fastener guide 14 can form various angles α2 between the distal end and the proximal end of the guide 14. For example, angle α2 can be in the range of 15 to 60 degrees, or about 40 degrees.

Fig. 3 shows a cross-sectional view of the elongate cannula 12 and attachment shaft 18 of the insertion tool 10. The attachment shaft 18 extends through and along the entire length of the elongate cannula 12. The handle portion 22 of the shaft 18 fits like a sleeve over the proximal end of the shaft 18 such that one end of the handle 22 sits flush against the proximal end of the cannula 12. At the distal end, the attachment shaft 18 tapers down, and forms an attachment fitting or means 30 for mating to an implant device. The attachment means 30 protrudes from the connector 24 enough to be inserted in an implant device, wherein the implant device can fit flush against the flat face of the connector 24. The flat face of the connector 24 is further shown in Fig. 4. Attachment means 30 is positioned between the distal end openings 17a, 17b of fastener guides 14a, 14b. Connector 24 has recessed cavities shaped to fit the distal ends of the fastener guides 14a, 14b. The recessed cavities of connector 24 are curved to form a circular surface that mates with cut-out portions at the distal ends of the fastener guides 14a, 14b. The curved circular surface of the recessed cavities mate with the distal ends of the guides to form a smooth and seam-free connection such that the recessed portions of connector 24 form a portion of the guide openings 17a, 17b. Thus, the recessed cavities are angled at the same degree as the distal ends of the guides 14a, 14b.

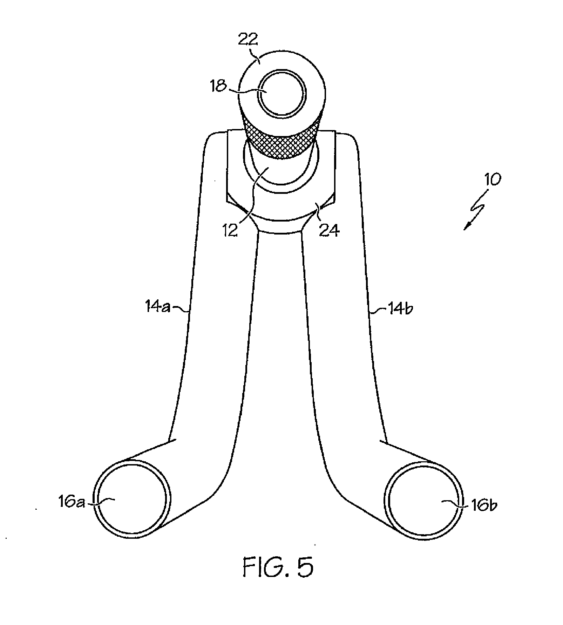

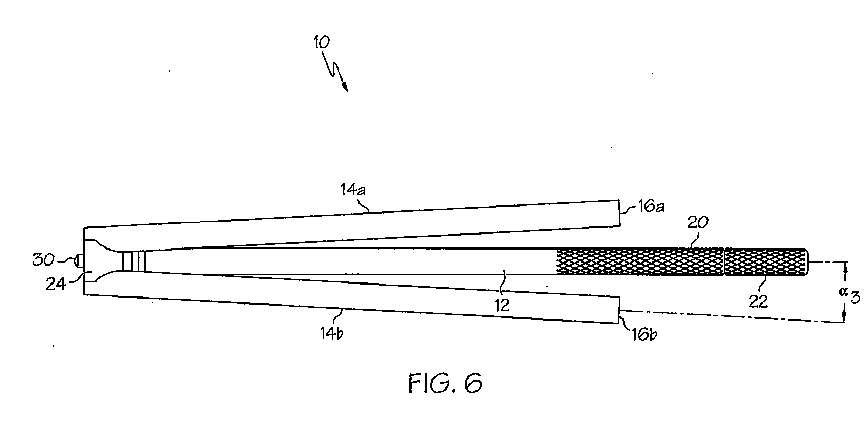

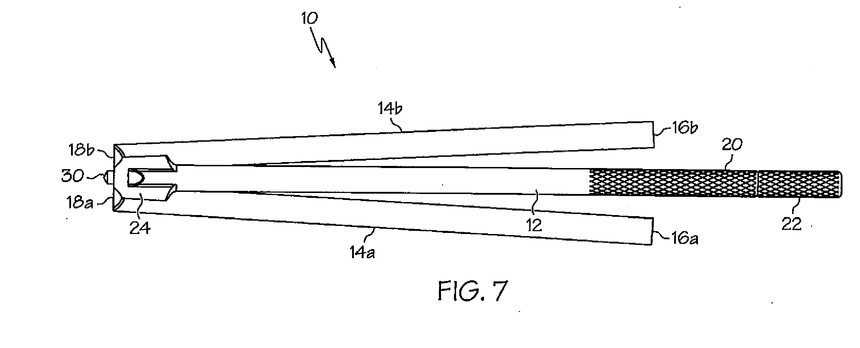

Figs. 5 through 7 show various views of an insertion tool 10. Fig. 5 is a rear view of an insertion tool 10. The proximal end openings 16a, 16b of fastener guides 14a, 14b are circular and can accommodate a cylindrical fastener driver or drill. The fastener guides 14a, 14b curve upward towards connector 24 where the distal ends of the guides 14a, 14b are coupled to the elongate cannula 12 and attachment shaft 18 by the connector 24 and form angle α1. Figs. 6 and 7 respectively show a bottom and top view of an insertion tool 10. The fastener guides 14a, 14b extend from connector 24 towards the proximal end of the attachment shaft 18, and form an angle, α3, relative to the length-wise axis of the attachment shaft 18. For example, angle α3 can be in the range of 0 to 20 degrees, or about 3 degrees. Angle α3 can be minimized to accommodate a small incision in the patient's skin. Angle α3 can further be adjusted to allow a user to insert bone fasteners into a bone body at an angle other than angle α1 discussed above. For example, angle α3 can let the user "toe in" bone fasteners into a bone body to resist backout of the bone fasteners or expulsion of the implant device 40.

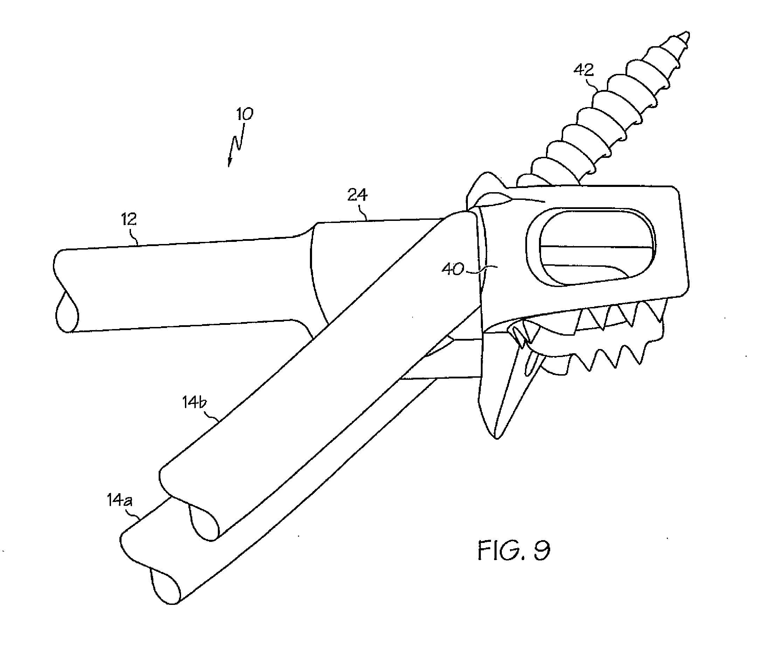

Figs. 8 and 9 show a side perspective view of an insertion tool 10 having an implant device 40 attached thereto. The implant device 40 fits flush and tightly against the flat face of the connector 24. Beneficially, a flush fit between the implant device 40 and connector 24 minimizes the risk of tissue damage, such as that caused by pinching or catching of tissue between the implant device 40 and connector 24. Although not visible, the implant device 40 is docked to the distal end of the attachment shaft 18 protruding outward from the flat face of the connector 24. Fastener guides 14a, 14b are aligned with fastener holes in the implant device 40. As shown, a bone fastener 42, in the form of a bone screw, extends through a fastener hole in the implant device 40. The bone fastener 42 is positioned at an angle, which corresponds to angle α1 formed by the distal end of the fastener guide 14b with respect to the elongate cannula 12 and attachment shaft 18. In use, the bone fastener 42 can be inserted in the opening in the proximal end of the fastener guides 14a, 14b, forced through the guides and out the opening in the distal end that is aligned with the fastener hole in the implant device. The bone fastener 42 can be driven into a bone body by the use of a flexible driver that can be inserted through the fastener guides.

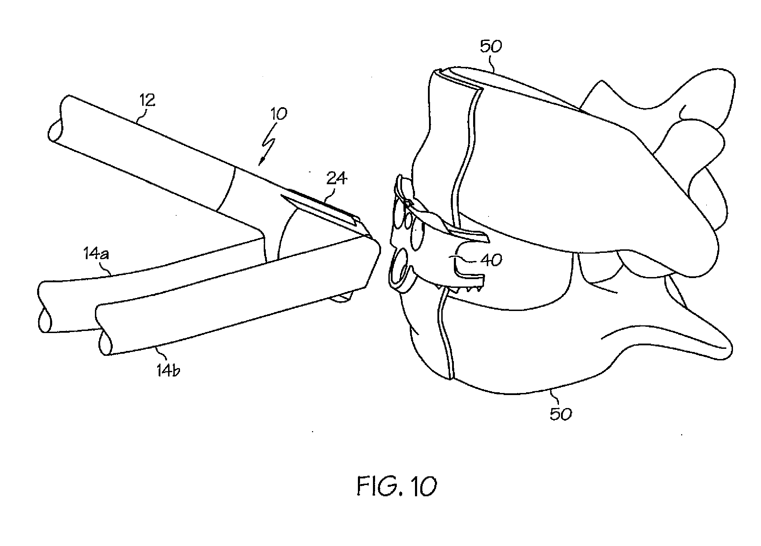

Figure 10 illustrates an implant device 40 being inserted at a surgical site with the use of an insertion tool 10. The implant device 40 is configured to fix and secure two or more bone bodies. The implant device 40 can fix and secure adjacent bone bodies (i.e., vertebrae) 50 of the spine that have had cartilaginous disc between the vertebrae replaced with material that promotes the fusion of the vertebrae such as a graft of bone tissue or some other similar material. The implant device 40 can have angled fastener holes. By being angled, each bone fastener 42 is positioned along an angle of rotation of a corresponding bone body 50 as well as an angle of settling of the bone body 50. This configuration places each fastener 42 in a protected position against motion of the spinal column. As a result, significant shear forces are not exerted on the fasteners 42 as the vertebral bodies rotate and/or settle.

Once the implant device 40 is inserted between two bone bodies 50, and the bone fasteners 42 are inserted through the fastener guides 14a, 14b and driven into the bone bodies 50, the insertion tool 10 can be detached from the implant device 40. For example, if the attachment shaft 18 is docked to the implant device 40 by means of a threaded connection, the attachment shaft 18 can be rotated in the elongate cannula 12 and connector through hole to unscrew the distal end of the shaft 18 from the implant device 40. Various other ways of docking and detaching the attachment shaft 18 and/or attachment means 30 to the implant device 40 can be used. For example, a bayonet lock, spring plunger, expandable pin or other interface components can be used. After being detached, the insertion tool 10 can be removed from the patient's body cavity through the incision in the skin.

In one embodiment, depending of the method of docking the implant device 40 to the attachment shaft 18 and/or attachment means 30, the insertion tool 10 may not include the elongate cannula 12 as discussed above (not shown). For example, the insertion tool 10 can include a fastener guide 14a, 14b and an attachment shaft 18, wherein the attachment shaft 18 can optionally have attachment means 30 at the distal end of the attachment shaft 18, and the insertion tool 10 does not have an elongate cannula 12. In this embodiment, as discussed above, various ways of docking the attachment shaft 18 and/or attachment means 30 to the implant device 40 can be used. The attachment shaft 18 can be coupled to the fastener guides 14a, 14b by connector 24. The connector 24 can be permanently secured to the attachment shaft 18 by convention means, such as by welding or with an adhesive. The connector 24 can have a through hole for receiving the attachment shaft 18 such that the attachment shaft 18 extends entirely through the connector 24, with a portion of the attachment shaft 18, or the attachment means 30 connected to the distal end of the attachment shaft 18, protruding from a face of the connector 24. The portion of the attachment shaft 18 protruding from the face of the connector 24, at the distal end of the insertion tool 10, can be docked to the implant device 40. In use, the implant device 40 can be docked to the insertion tool 10 at the portion of the attachment shaft 18 protruding out of the connector 24, and then the insertion tool 10 can be used to locate the implant device 40 at the surgical site. Fastener tools and bone fasteners can be inserted into the proximal openings in the fastener guides 14a, 14b to fix the implant device 40 to a bone body, as discussed above. The insertion tool 10 can then be detached from the implant device 40 and the insertion tool 10 can be retracted out of the patient's body. In the case the insertion tool 10 does not include the elongate cannula 12, and the attachment shaft 18 is permanently secured to the connector 24, the method of docking the implant device 40 to the portion of the attachment shaft 18 protruding from the connector 24 is ideally suited to the use of non-threaded connections that may require rotating the attachment shaft 18 to detach it from the implant device 40. In the case a threaded-type of connection is desired for docking the implant device 40 to the insertion tool 10, the elongate cannula 12 can be used to accommodate rotation of the attachment shaft 18.

While shown embodiments of the present invention are described for inserting implant devices and bone fasteners for securing such devices to a bone body at the surgical site, persons skilled in the art would recognize that the insertion tool of the present invention may be utilized to insert implant devices for support in adjoining cervical, thoracic and lumbar in the region of the vertebral body. Further, the insertion tool and guide system of the invention is not limited to vertebral bodies and associated implant devices, but can also be used with any implant device to join two other pieces of bone in other parts of the body. Similarly, although the insertion tool 10 is shown to have one or two fastener guides, the tool can have any number of fastener guides depending on the implant device to be inserted and the number of fastener holes therein.

While embodiments and applications of this invention have been shown and described, it would be apparent to those skilled in the art that many more modifications are possible without departing from the inventive concepts herein. The invention, therefore, is not to be restricted except in the spirit of the appended claims.

## Claims

1. An insertion tool (10) including:
an elongate cannula (12) having a proximal end and a distal end,
a fastener guide (14a; 14b) having a proximal end and a distal end, said distal end of said fastener guide (14a; 14b) being coupled to said distal end of said elongate cannula (12), wherein a portion of said fastener guide (14a; 14b) is curved; and
said fastener guide (14a; 14b) having an opening (16a; 16b) at said proximal end for receiving a bone fastener (42).

2. The insertion tool (10) of claim 1, said bone fastener (42) being attachable to a driver device.

3. The insertion tool (10) according to any of the preceding claims, said elongate cannula (12) having a passage there through for receiving an attachment shaft (18).

4. The insertion tool (10) of claim 3, said attachment shaft (18) having a proximal end and a distal end, wherein said attachment shaft (18) has attachment means (30) secured to said distal end of said attachment shaft (18) for attaching an implant device (40) to said distal end of said attachment shaft (18).

5. The insertion tool (10) according to any one of claims 3 to 4, said attachment shaft (18) being rotatable within said elongate cannula (12).

6. The insertion tool (10) according to any one of the preceding claims, further including a connector (24) for coupling said distal end of said fastener guide (14a; 14b) to said distal end of said elongate cannula (12).

7. The insertion tool (10) of claim 6, said distal end of said fastener guide (14a; 14b) being coupled at an angle relative to said elongate cannula (12), said angle being in the range of 15 to 60 degrees.

8. The insertion tool (10) of claim 7, said proximal end of said fastener guide (14a; 14b) being substantially parallel with said proximal end of said elongate cannula (12).

9. The insertion tool (10) of claim 6, when depending on claim 3, said connector (24) including a through hole for receiving said attachment shaft (18).

10. The insertion tool (10) of claim 9, when depending on claim 4, wherein a portion of said attachment means (30) extends outward beyond said through hole in said connector (24).

11. The insertion tool (10) according to any one claims 3 to 10, said attachement shaft (18) being straight and rigid.

12. The insertion tool (10) according to any one of the preceding claims, said fastener guide (14a; 14b) having a passage there through for receiving a fastener tool.

13. The insertion tool (10) according to any one of claims 3 to 12, said fastener guide (14a; 14b) having a length and said attachment shaft (18) having a length, and said length of said fastener guide (14a; 14b) being at least 50% of said length of said attachment shaft (18).

14. The insertion tool (10) according to any one of claims 3 to 13, said attachment shaft (18) having a proximal end and a distal end, wherein said proximal end of said attachment shaft (18) and said proximal end of said fastener guide (14a; 14b) are dimensioned and configured to be outside of a patient's body during an anterior approach surgical procedure on the spine.

15. A guide system comprising the insertion tool (10) according to any of the preceding claims and an implant device (40), the system being arranged for implanting the implant device (40) having at least one fastener hole, wherein:
said distal end of said fastener guide (14a; 14b) being aligned with said fastener hole in said implant device (40);
said elongate cannula (12) having a passage there through for receiving an attachment shaft (18); and
said attachment shaft (18) extending through said passage in said elongate cannula (12), said attachment shaft (18) being docked to said implant device (40).
